# NEW EUROPEAN PATENT SPECIFICATION

(11) **EP 0 716 884 B2**
(45) Date of publication and mention of the opposition decision: **23.09.2009**
(45) Mention of the grant of the patent: 21.03.2001
(21) Application number: 95203469.2
(22) Date of filing: 13.12.1995
(51) Int. Cl.: B01J 23/66, C07D 301/10

(54) **Process for preparing ethylene oxide catalysts**
Verfahren zur Herstellung von Äthylenoxid-Katalysatoren
Procédé de préparation de catalyseurs pour l'oxide d'éthylène

(30) Priority: 15.12.1994 US 357620; 11.05.1995 US 439512; 15.12.1994 US 357621
(43) Date of publication of application: 19.06.1996
(73) Proprietor: SHELL INTERNATIONALE RESEARCH MAATSCHAPPIJ B.V., 2596 HR Den Haag (NL)
(72) Inventor: Kemp, Richard A., Stafford, Texas 77477 (US); Evans, Wayne E., Richmond, Texas 77469 (US); Matusz, Marek, Houston, Texas 77084 (US)

(56) References cited:
- EP-A- 0 207 550
- EP-A- 0 244 895
- EP-A- 0 266 015
- EP-A- 0 315 911
- JP-A- 04 346 835
- US-A- 4 007 135
- US-A- 4 207 210
- US-A- 4 916 243
- US-A- 5 102 848

## Description

The invention relates to a process for the preparation of silver-containing catalysts suitable for the preparation of ethylene oxide.

Catalysts for the production of ethylene oxide from ethylene and molecular oxygen are generally supported silver catalysts. Such catalysts are typically promoted with alkali metals. Small amounts of the alkali metals potassium, rubidium and cesium were noted as useful promoters in supported silver catalysts in U.S. Patent No. 3,962,136, issued June 8, 1976, and U.S. Patent No. 4,010,115, issued March 1, 1977. The use of other co-promoters, such as rhenium, or rhenium along with sulphur, molybdenum, tungsten and chromium is disclosed in U.S. Patent No. 4,766,105, issued August 23, 1988, and U.S. Patent No. 4,808,738, issued February 28, 1989. U.S. Patent No. 4,908,343, issued March 13, 1990, discloses a supported silver catalyst containing a mixture of a cesium salt and one or more alkali metal and alkaline earth metal salts.

In EP-A 0 207 550 there is disclosed a process for preparing an ethylene oxide production catalyst by applying silver to a carrier, in which process the carrier has been prepared by mixing an aluminium compound with a salt of a metal of Group 1^{A} of the periodic system, in particular potassium, rubidium and cesium, and by calcining the obtained mixture.

In EP-A 0 244 895 there is disclosed an ethylene oxide production silver- containing catalyst which is also based on a calcined, alkali metal-enriched carrier and which is further characterised by the addition of an alkali metal and a fluoride anion to the catalyst composition.

In EP-A 0 315 911 there is disclosed a silver-deposited ethylene oxide production catalyst comprising a carrier composed mainly of alpha-alumina, which carrier does not show acidity when using a dimethyl yellow indicator having a pKa of +3.3 and does not show basicity when using a bromothymol blue indicator having a pKa of +7.1. According to the specification the indicated acid/base balance of the carrier is achieved by controlling the amount of silica and sodium or potassium, respectively, contained therein. In the examples an alpha-alumina carrier containing both silica and sodium is immersed in a solution of sodium bicarbonate, heated with superheated steam at 150°C and subsequently impregnated with the other catalyst components.

In US-A 4,207,210 there is disclosed a process for preparing an ethylene oxide production catalyst comprising pre-impregnating a porous refractory support with an aqueous solution of a salt of a higher alkali metal selected from potassium, rubidium, cesium and mixtures thereof, at least partly drying the support at 100-200°C and subsequently impregnating the pre-impregnated support with a liquid phase containing silver. In the specification it is stated that the purpose of pre-impregnation is to improve selectivity but that the lower alkali metals, lithium and sodium, are ineffective for this purpose.

It has now been found that catalysts pre-doped, pretreated or pre-impregnated with a promoting amount of potassium have improved selectivity and/or selectivity stabilities relative to those obtained with catalysts which have not been thus pre-doped.

The invention therefore relates to a process for the preparation of a catalyst for the production of ethylene oxide from ethylene and molecular oxygen in the vapour phase which process comprises depositing a predopant amount of potassium on a shaped porous, refractory support, drying the support, and thereafter depositing a catalytically effective amount of silver, a promoting amount of alkali metal, a promoting amount of rhenium, and optionally a promoting amount of a rhenium co-promoter selected from sulphur, molybdenum, tungsten, chromium, phosphorus, boron and mixtures thereof, on the support, and subsequently drying the support.

Generally, in the vapour phase reaction of ethylene with oxygen to produce ethylene oxide, the ethylene is present in at least a double amount (on a molar basis) compared with oxygen, but frequently is often much higher. Therefore, the conversion is calculated according to the mole percentage of oxygen which has been consumed in the reaction to form ethylene oxide and any oxygenated by-products. The oxygen conversion is dependent on the reaction temperature, and the reaction temperature is a measure of the activity of the catalyst employed. The value T₄₀ indicates the temperature at 40 mole percent oxygen conversion in the reactor and is expressed in °C. This temperature for any given catalyst is higher when the conversion of oxygen is higher. Moreover, this temperature is strongly dependent on the employed catalyst and the reaction conditions. The selectivity (to ethylene oxide) indicates the molar amount of ethylene oxide in the reaction product compared with the total molar amount of ethylene converted. In this specification, the selectivity is indicated as S₄₀, which means the selectivity at 40 mole percent oxygen conversion, and the activity is indicated as T₄₀. The selectivity of the catalyst is important because it determines how efficiently the process operates. For example, for the highest efficiency, it is desired to minimize undesired side reactions of the process by utilizing a catalyst with high selectivity. Also important to overall efficiency, and hence, economics, is the stability of the selectivity of the catalyst. It is desired that the catalyst maintain its selectivity over a long period of time, and any improvements in extending the useful lifetime of the catalyst by maintaining a higher level of selectivity are extremely important in operating efficiency and economics.

The catalysts prepared according to the instant invention comprise a catalytically effective amount of silver, a promoting amount of alkali metal, a promoting amount of rhenium, and, optionally, a promoting amount of a rhenium co-promoter selected from sulphur, chromium, molybdenum, tungsten, phosphorus, boron and mixtures thereof, supported on a porous, refractory support which has been pretreated with a pre-doping amount of potassium.

In general, the catalysts prepared according to the present invention are prepared by impregnating shaped porous refractory supports with potassium dissolved in a suitable solvent sufficient to cause deposition on the support of from 10 to 5000, preferably from 15 to 500 parts per million by weight of the total catalyst, of potassium predopant. The porous refractory support is then dried and thereafter impregnated with silver ions or compound(s), complex(es) and/or salt(s) dissolved in a suitable solvent sufficient to cause deposition on the support of from 1 to 40, preferably from 1 to 25 percent by weight, basis the weight of the total catalyst, of silver. The impregnated support is subsequently separated from the solution and the deposited silver compound is reduced to metallic silver. Also deposited on the support either prior to, coincidentally with, or subsequent to the deposition of the silver will be suitable ions, or compound(s) and/or salt(s) of alkali metal dissolved in a suitable solvent. Also deposited on the support either prior to, coincidentally with, or subsequent to the deposition of the silver and/or alkali metal will be suitable rhenium ions or compound(s), complex(es) and/or salt(s) dissolved in an appropriate solvent. Optionally deposited on the support either prior to, coincidentally with, or subsequent to the deposition of the silver and/or alkali metal and/or rhenium will be suitable ions or salt(s), complex(es) and/or compound(s) of sulphur, molybdenum, tungsten, chromium phosphorus and/or boron dissolved in an appropriate solvent.

The carrier or support employed in these catalysts in its broadest aspects can be any of the large number of conventional, porous refractory catalyst carriers or support materials which are considered relatively inert in the presence of ethylene oxidation feeds, products and reaction conditions. Such conventional materials are known to those skilled in the art and may be of natural or synthetic origin and preferably are of a macroporous structure, i.e., a structure having a surface area below about 10 m²/g and preferably below about 3 m²/g. Particularly suitable supports are those of aluminous composition though other materials such as charcoal, pumice, magnesia, zirconia, kieselguhr, fuller's earth, silicon carbide, porous agglomerates comprising silica and/or silicon carbide, silica, magnesia, selected clays, artificial and natural zeolites and ceramics can also be used as supports. Refractory supports especially useful in the preparation of catalysts in accordance with this invention comprise the aluminium oxide, in particular those comprising alpha alumina. In the case of alpha alumina-containing supports, preference is given to those having a specific surface area as measured by the B.E.T. method of from 0.03 to 10, preferably from 0.05 to 5, more preferably from 0.1 m²/g to 3 m²/g, and a water pore volume as measured by conventional water absorption techniques of from 0.1 to 0.75 ml/g by volume. The B.E.T. method for determining specific surface area is described in detail in Brunauer, S., Emmet, P. Y. and Teller, E., J. Am. Chem. Soc., 60, 309-16 (1938).

The support is shaped into particles, chunks, pieces, pellets, rings, spheres, wagon wheels, and the like of a size suitable for use in fixed bed reactors. Conventional commercial fixed bed reactors are typically in the form of a plurality of parallel elongated tubes (in a suitable shell) filled with catalyst. In such reactors, it is desirable to use a support formed into a rounded shape, such as, for example, spheres, pellets, rings, tablets and the like, having diameters from about 2.5 mm to about 20 mm.

The catalysts are prepared by a technique in which potassium predopant in the form of soluble salts and/or compounds is deposited on the support which is then subjected to drying or to a thermal treatment sufficient to allow deposition of the potassium salts and, while not wishing to be bound by any particular theory, presumed complexation with the anionic components on the surface of the catalyst. Thereafter, an alkali metal promoter(s), the rhenium promoter, and the rhenium co-promoter, if present, in the form of soluble salts and/or compounds are deposited on the catalyst and/or support prior to, simultaneously with, or subsequent to the deposition of the silver and each other. The preferred method is to deposit silver, alkali metal promoter, rhenium and rhenium co-promoter, if present, simultaneously on the support, that is, in a single impregnation step. Potassium however must be deposited on the support prior to all of the other catalyst components and the support must then be dried to a degree sufficient to fix the alkali metal on the support before deposition of the other catalyst components.

Generally, the carrier is contacted with potassium predopant in salt, compound or potassium complex form which has been dissolved in an aqueous or essentially aqueous solution, dried at a temperature in the range of from 200 °C to 1000 °C, and then contacted with silver in salt, compound, or complex form which has been dissolved in an aqueous solution, so that the carrier is impregnated with said aqueous solution; thereafter the impregnated carrier is separated form the aqueous solution, e.g., by centrifugation or filtration and then dried. The thus obtained impregnated carrier is heated to reduce the silver to metallic silver. It is conveniently heated to a temperature in the range of from 50 °C to 600 °C, during a period sufficient to cause reduction of the silver salt, compound or complex to metallic silver and to form a layer of finely divided silver, which is bound to the surface of the carrier, both the exterior and pore surface. Air, or other oxidizing gas, reducing gas, an inert gas or mixtures thereof may be conducted over the carrier during this heating step.

Although alkali metals do exist in a pure metallic state, they are not suitable for use in that form. The alkali metals are used as an ion or compound dissolved in a suitable solvent for impregnation purposes.

In a preferred embodiment, the potassium compound is selected from the group consisting of potassium acetate, nitrate, chloride, oxide, hydroxide, oxalate and mixtures thereof, with potassium nitrate, oxalate, acetate and mixtures thereof being particularly preferred. Particularly preferred potassium promoters are potassium nitrate and potassium acetate. In a preferred embodiment, the lithium compound is selected from the group consisting of lithium hydroxide, oxide, chloride, carbonate, acetate, oxalate and mixtures thereof, with lithium hydroxide, oxide, acetate and mixtures thereof being particularly preferred. Particularly preferred lithium promoters are lithium hydroxide and lithium acetate.

In a preferred embodiment, the cesium compound is selected from the group consisting of cesium acetate, acetylacetonate, chloride, carbonate, nitrate, oxalate, hydroxide and mixtures thereof, with cesium nitrate, acetate, chloride and mixtures thereof being particularly preferred. Particularly preferred cesium promoters are cesium nitrate and cesium acetate.

Promoting amounts of at least one alkali metal promoter(s) are deposited on the pre-doped support using a suitable solution. The amount of alkali metal promoter deposited upon the pre-doped support generally lies between 10 and 5000, preferably between 15 and 2000 and more preferably, between 20 and 1500 parts per million by weight of total catalyst. Most preferably, the amount ranges between 50 and 1000 parts per million by weight of the total catalyst.

The alkali metal promoters are present on the catalysts in the form of cations or compounds of complexes or surface compounds or surface complexes rather than as the extremely active free alkali metals, although for convenience purposes in this specification and claims they are referred to as "alkali metal" or "alkali metal promoters". For purposes of convenience, the amount of alkali metal deposited on the support or present on the catalyst is expressed as the metal.

In a preferred embodiment, at least a major proportion (greater than 50%) of the alkali metal promoters comprise the higher alkali metals. As used herein, the term "higher alkali metal" and cognates thereof refers to the alkali metals selected from the group consisting of potassium, rubidium, cesium and mixtures thereof. A preferred alkali metal promoter is cesium. A particularly preferred alkali metal promoter is cesium plus at least one additional alkali metal. The additional alkali metal is preferably selected from sodium, lithium and mixtures thereof, with lithium being preferred.

The carrier is also impregnated with rhenium ions, salt(s), compound(s), and/or complex(es). This may be done at the same time that the alkali metal promoter is added, or before or later; or at the same time that the silver is added, or before or later; or at the same time that the rhenium co-promoter, if present, is added, or before or later. Preferably, rhenium, alkali metal, rhenium co-promoter, if present, and silver are in the same impregnating solution. The preferred amount of rhenium, calculated as the metal, deposited on or present on the carrier or catalyst ranges from 0.1 to 10, more preferably from 0.2 to 5 micromoles per gram of total catalyst, or, alternatively stated, from 19 to 1860, preferably from 37 to 930 parts per million by weight of total catalyst.

Suitable rhenium compounds for use in the preparation of the instant catalysts are rhenium compounds that can be solubilized in an appropriate solvent. Preferably, the solvent is a water-containing solvent. More preferably, the solvent is the same solvent used to deposit the silver and the alkali metal promoter. Examples of suitable rhenium compounds include the rhenium salts such as rhenium halides, oxyhalides, rhenates, perrhenates, oxides and acids. A preferred compound for use in the impregnation solution is the perrhenate, preferably ammonium perrhenate. However, the alkali metal perrhenates, alkaline earth metal perrhenates, silver perrhenates, other perrhenates and rhenium heptoxide can also be suitably utilized. In a preferred embodiment of the instant invention, the rhenium present on the catalyst is present in a form that is extractable in a dilute aqueous base solution.

U.S. Patent No. 4,766,105 teaches that if a rhenium co-promoter is added to an alkali metal/rhenium doped supported silver catalyst, an improvement in initial selectivity is obtained. While suitable catalysts can be prepared in the absence of a rhenium co-promoter, it is preferable that the catalysts contain a rhenium co-promoter. When a co-promoter is utilized, it is selected from the group consisting of (preferably a compound of) sulphur, molybdenum, tungsten, chromium, phosphorus, boron and mixtures thereof. In a presently preferred embodiment, the co-promoter is applied to the catalyst in the oxyanionic form. Preferred are sulfates, molybdates, tungstates, chromates, phosphates and borates. The anions can be supplied with various counter-ions. Preferred are ammonium, alkali metal, mixed alkali metal and hydrogen (i.e. acid form).

The preferred amount of rhenium co-promoter compound present on or deposited on the support or catalyst ranges from 0.1 to 10, preferably from 0.2 to 5 micromoles, expressed as the element, per gram of total catalyst.

One method of preparing the silver containing catalyst can be found in U.S. Patent 3,702,259. Other methods for preparing the silver-containing catalysts which in addition contain higher alkali metal promoters can be found in U.S. Patent 4,010,115; U.S. Patent 4,356,312; U.S. Patent 3,962,136, and U.S. Patent 4,012,425. Methods for preparing silver-containing catalysts containing higher alkali metal and rhenium promoters can be found in U.S. Patent No. 4,761,394, and methods for silver-containing catalysts containing higher alkali metal and rhenium promoters and a rhenium co-promoters selected from sulphur, chromium, molybdenum and/or tungsten can be found in U.S. Patent No. 4,766,105.

In general terms, the impregnation process comprises pre-impregnating the support with one or more solutions comprising potassium drying the impregnated support, impregnating the thus pre-doped support with one or more solutions comprising silver, alkali metal promoter, rhenium, and optionally rhenium co-promoter, and drying the support. As used in the instant specification and claims, the terminology "impregnating the support with one or more solutions comprising silver, alkali metal, rhenium and/or rhenium co-promoter", and similar or cognate terminology means that the support is impregnated in a single or multiple impregnation with one solution containing silver, alkali metal, rhenium and rhenium co-promoter in differing amounts; or in multiple impregnations with two or more solutions, wherein each solution contains at least one component selected from silver, alkali metal, rhenium and rhenium co-promoter, with the proviso that all of the components will individually be found in at least one of the solutions. For purposes of the pre-impregnation step, the concentration of the Group I element (expressed as the element) will range from 1 x 10⁻⁵ up to 1 and preferably, from 5 x 10⁻⁴ to 0.1 g/l when a single pre-impregnation step is utilized. In the impregnation step the concentration of the silver (expressed as the metal) in the silver-containing solution will range from 1 g/l up to the solubility limit when a single impregnation is utilized. The concentration of the alkali metal promoter (expressed as the metal) will range from 1 x 10⁻³ up to 12 and preferably, from 10 x 10⁻³ to 12 g/l when a single impregnation step is utilized. The concentration of the rhenium (expressed as the metal) will range from 5 x 10⁻³ to 20 and preferably from 50 x 10⁻³ to 20 g/l when a single impregnation step is utilized. The concentration of rhenium co-promoter (expressed as the element), if present, will range from 1 x 10⁻³ to 20 and preferably from 10 x 10⁻³ to 20 g/l when a single impregnation step is utilized. Concentrations selected within the above noted ranges will depend upon the pore volume of the catalyst, the final amount desired in the final catalyst and whether the impregnation is single or multiple. Appropriate concentrations can be readily determined by routine experimentation.

The amount of silver deposited on the support or present on the support during impregnation is to be a catalytically effective amount of silver, i.e., an amount that catalyzes the reaction of ethylene and oxygen to produce ethylene oxide. Preferably this amount will range from 1 to 40, more preferably from 1 to 25, and even more preferably from 5 to 20 percent by weight of the total catalyst.

The silver catalysts prepared according to the present invention have been shown to have high initial selectivity and/or high selectivity stabilities for ethylene oxide production in the direct oxidation of ethylene with molecular oxygen to ethylene oxide. The conditions for carrying out such an oxidation reaction in the presence of the silver catalysts according to the present invention broadly comprise those already described in the prior art. This applies, for example, to suitable temperatures, pressures, residence times, diluent materials such as nitrogen, carbon dioxide, steam, argon, methane or other saturated hydrocarbons, to the presence of moderating agents to control the catalytic action, for example, 1,2-dichloroethane, vinyl chloride, ethyl chloride or chlorinated polyphenyl compounds, to the desirability of employing recycle operations or applying successive conversations in different reactors to increase the yields of ethylene oxide, and to any other special conditions which may be selected in processes for preparing ethylene oxide. Pressures in the range of from atmospheric to about 3500 kPa are generally employed. Higher pressures, however, are not excluded. Molecular oxygen employed as reactant can be obtained from conventional sources. The suitable oxygen charge may consist essentially or relatively pure oxygen, a concentrated oxygen stream comprising oxygen in major amount with lesser amounts of one or more diluents, such as nitrogen and argon, or another oxygen-containing stream, such as air. It is therefore evident that the use of the catalysts according to the present invention in ethylene oxide reactions is in no way limited to the use of specific conditions among those which are known to be effective. For purposes of illustration only, the following table shows the range of conditions that are often used in current commercial ethylene oxide reactor units.

**TABLE I**

| | |
|---|---|
| *GHSV | 1500-10,000 |
| Inlet Pressure | 1200-3000 kPa |

| Inlet Feed | |
|---|---|
| Ethylene | 1-40% |
| O₂ | 3-12% |
| Ethane | 0-3% |
| Argon and/or methane and/or nitrogen diluent | Balance |
| Chlorohydrocarbon Moderator | 0.3-50 ppmv total |
| Coolant temperature | 180-315 °C |
| Catalyst temperature | 180-325 °C |
| O₂ conversion level | 10-60% |
| EO Production (Work Rate) | 32-56 kg EO/m³ of catalyst/hr |

| | |
|---|---|
| * Volume units of gas at standard temperature and pressure passing over one volume unit of packed catalyst per hour. | |

In a preferred application of the silver catalysts prepared according to the present invention, ethylene oxide is produced when an oxygen-containing gas is contacted with ethylene in the presence of the present catalysts at a temperature in the range of from about 180 °C to about 330 °C and preferably 200 °C to 325 °C.

The following illustrative embodiments, are not of the invention but are illustrative of the preparation and effects of the invention.

### Illustrative Embodiments

### Illustrative Embodiment 1

### Part A: Preparation of stock silver oxalate/ethylenediamine solution for use in catalyst preparation:

1) Dissolve 415 grams (g) of reagent-grade sodium hydroxide in 2340 millilitres (ml) deionized water. Adjust the temperature to 50 °C.
2) Dissolve 1699 g of "Spectropure" (high purity) silver nitrate in 2100 ml deionized water. Adjust the temperature to 50 °C.
3) Add sodium hydroxide solution slowly to silver nitrate solution with stirring while maintaining a temperature of 50 °C. Stir for 15 minutes after addition is complete, and then lower the temperature to 40 °C.
4) Insert clean filter wands and withdraw as much water as possible from the precipitate created in step (3) in order to remove sodium and nitrate ions. Measure the conductivity of the water removed and add back as much fresh deionized water as was removed by the filter wands. Stir for 15 minutes at 40 °C. Repeat this process until the conductivity of the water removed is less than 90 µmho/cm. Then add back 1500 ml deionized water.
5) Add 630 g of high-purity oxalic acid dihydrate in approximately 100 g increments. Keep the temperature at 40 °C and stir to mix thoroughly. Add the last portion of oxalic acid dihydrate slowly and monitor pH to ensure that pH does not drop below 7.8.
6) Remove as much water from the mixture as possible using clean filter wands in order to form a highly concentrated silver-containing slurry. Cool the silver oxalate slurry to 30 °C.
7) Add 699 g of 92 percent weight (%w) ethylenediamine (8% deionized water). Do not allow the temperature to exceed 30 °C during addition.

The above procedure yields a solution containing approximately 27-33%w silver.

### Part B: Catalyst pre-doping procedure:

For Catalyst A, in order to deposit 14 parts per million (ppm) (2.0 micromoles/gram) of lithium ions, the following solution was made. 30 ml of monoethanolamine was diluted with 570 ml of deionized water to yield a total volume of 600 ml. Lithium hydroxide monohydrate (0.1083 g) was dissolved in the above solution. 400 g of Catalyst carrier I (99 wt% alpha alumina, B.E.T. surface area 0.48 m²/g, water pore volume 0.465 ml/g) was then vacuum impregnated at 3.33-6.66 kPa vacuum for three minutes. At the end of this time, the vacuum was released and the excess solution decanted from the carrier. The carrier was then dried by continuous shaking in an 8500 litre/hr air stream at 270 °C for seven minutes, followed by calcination in a flowing air oven at 600 °C for four hours.

For Catalyst B, the above procedure was repeated, except that potassium nitrate (0.2764 g) (4 micromoles/g) was used in place of the lithium hydroxide monohydrate in order to deposit 156 ppm of potassium ions on the support.

For Catalyst C, no pre-doping of Catalyst carrier I was done.

For Catalyst D, in order to deposit 14 parts per million (ppm) (2.0 micromoles/gram) of lithium ions, the following solution was made. 12.5 Grams of monoethanolamine was diluted with 237.5 grams of deionized water to yield a total weight of 250 grams. Lithium hydroxide monohydrate (0.0548 g) was dissolved in the above solution. 150 g of Catalyst carrier II (Alpha alumina 98.5, B.E.T. surface area 0.51 m²/g, water pore volume 0.38 ml/g) was then vacuum impregnated at 3.33-6.66 kPa vacuum for three minutes. At the end of this time, the vacuum is released and the excess solution is decanted from the carrier. The carrier is then dried by continuous shaking in a 8500 litre/hr air stream at 270 °C for seven minutes, followed by calcination in a flowing air oven at 600 °C for four hours.

For Catalyst E, no pre-doping of Catalyst carrier II was done.

For Catalyst F, in order to deposit 345 parts per million (ppm), basis the final catalyst, of cesium ions, the following solution was made. A stock aqueous cesium hydroxide solution containing 46.5 weight percent cesium was diluted by dissolving 0.463 grams of the stock solution into deionized water to a total weight of 200 grams. 40 Grams of Catalyst carrier III (Alpha alumina >98 wt%, B.E.T. surface area 0.68 ml/g, water pore volume 0.37 ml/g) is then vacuum impregnated at 3.33-6.66 kPa vacuum for three minutes. At the end of this time, the vacuum is released and the excess solution is decanted from the carrier. The carrier is then dried by continuous shaking in an 8500 litre/hr air stream at 270 °C, three minutes at 120-150 °C, and four minutes at 250 °C. This drying step is followed by calcination in air, taking approximately two hours to heat from room temperature to 700 °C. The temperature was maintained at 700 °C for four hours and thereafter cooled to room temperature over a period of about two hours.

For Catalyst G, no cesium pre-doping of Catalyst carrier III was carried out.

For Catalyst H, in order to deposit 500 parts per million (ppm) of cesium ions, the following solution was made. 50 g of monoethanolamine was diluted with 950 g of deionized water. 2.466 g of cesium hydroxide (50% solution) was mixed with the above solution. 600 grams of Catalyst carrier IV was then vacuum impregnated at 3.33-6.66 kPa vacuum for three minutes. At the end of this time, the vacuum was released and the excess solution is decanted from the carrier. The carrier was then dried by continuous shaking in an 8500 litre/hr air stream for ten minutes at 250 °C in 100 gram batches. The carrier was then calcined for four hours at 800 °C.

For Catalyst I, no pre-doping of Catalyst carrier IV was carried out.

### Part C: Preparation of impregnated catalysts:

For preparing impregnated Catalyst A, a solution was prepared by adding cesium hydroxide (0.1939 grams) to 350 g of the above-prepared silver oxalate/ethylenediamine solution (specific gravity = 1.553 g/ml). 50 Grams of the resulting solution was used to prepare a catalyst with a target cesium level of 315 ppm.

For Catalyst A, approximately 30 g of the lithium pre-doped carrier prepared above were placed under 3.33 kPa vacuum for 3 minutes at room temperature. Approximately 50 g of doped impregnating solution was then introduced to submerge the carrier, and the vacuum was maintained at 3.33 kPa for an additional 3 minutes. At the end of this time the vacuum was released, the excess impregnating solution was removed from the carrier by centrifugation for 2 minutes at 500 rpm. The impregnated carrier was then cured by being continuously shaken in an 8500 litre/hr air stream flowing across a cross-sectional area of approximately 19-32 cm² at 250 °C for 5 minutes. The cured catalyst was then ready for testing.

For Catalyst B, the above procedure was followed, except the potassium-doped carrier was used as the support. Additionally, cesium hydroxide (0.2424 grams) was added to 350 g of the above-prepared silver oxalate/ethylenediamine solution to give a target cesium level of 330 ppm.

For Catalyst C, the above procedure was used, except unmodified carrier I was used as the support. Additionally, cesium hydroxide (0.1873 grams) was added to 350 g of the above-prepared silver oxalate/ethylenediamine solution to give a target cesium level of 255 ppm.

The procedures set forth above for Catalysts A, B and C yielded catalysts on this carrier which contained approximately 13.5%w Ag with the following approximate dopant levels (expressed in parts per million by weight basis the weight of the total catalyst, i.e., ppmw) and which were approximately optimum in cesium for the given silver levels and support with regard to initial selectivity under the test conditions described below.

| | Pre-doped Potassium, ppmw | Pre-doped Lithium, ppmw | Cesium, ppmw |
|---|---|---|---|
| Catalyst A | -- | 14 | 315 |
| Catalyst B | 156 | -- | 330 |
| Catalyst C | -- | -- | 255 |

For each of Catalysts D and E, into a 10 ml beaker was added 0.3222 g of (NH₄)ReO₄ and approximately 3.0 g of ethylenediamine/H₂O (50/50 by weight), and the mixture allowed to dissolve with stirring. 0.1572 g of Li₂SO₄·4H₂0 was dissolved in 2 ml of water in a weighing dish, and then added to the perrhenate solution. 0.6777 g of LiNO₃ was dissolved in 3 ml of water and added to the perrhenate solution. The perrhenate/sulfate/nitrate solution was allowed to stir, ensuring complete dissolution. This solution was added to 366 g of the above-prepared silver solution (specific gravity = 1.476 g/ml), and the resulting solution diluted with water to a total weight of 402 g. 0.1872 g of aqueous CsOH solution (46.5% Cs) was added to a 100 g portion of the silver oxalate/dopant solution to prepare the final impregnation solution, which targeted a final catalyst cesium level of approximately 430 ppm. One-half of this cesium-doped solution was used to prepare a catalyst.

For Catalyst D, approximately 30 g of lithium pre-doped carrier II were placed under 3.33 kPa for 3 minutes at room temperature. Approximately 50 g of the above-prepared, doped impregnating solution was then introduced to submerge the carrier, and the vacuum maintained at 3.33 kPa for an additional 3 minutes. At the end of this time the vacuum was released, and the excess impregnating solution removed from the carrier by centrifugation for 2 minutes at 500 rpm. Then the impregnated carrier was cured by being continuously shaken in an 8500 litre/hr air stream flowing across a cross-sectional area of approximately 19-32 cm² at 260-270 °C for 6 minutes. The cured catalyst was then ready for testing.

For Catalyst E, the above procedure was followed except un-modified Catalyst carrier II was used.

The procedures set forth above for Catalysts D and E yielded catalysts on this carrier which contained approximately 13.5%w Ag with the following approximate dopant levels (expressed in parts per million by weight basis the weight of the total catalyst, i.e., ppmw) and which were approximately optimum in cesium for the given silver levels and support with regard to initial selectivity under the test conditions described below.

| Catalyst | Pre-doped Lithium, ppmw | Rhenium, ppmw | Cesium, ppmw |
|---|---|---|---|
| D | 14 | 279 | 430 |
| E | -- | 279 | 430 |

### Catalyst F (Rhenium-containing, pre-doped Cs)

For preparing impregnated Catalyst F, into a 10 ml beaker was added 0.168 g of (NH₄)ReO₄ and approximately 3 g of deionized water, and the mixture allowed to dissolve with stirring. 0.080 g of Li₂SO₄.H₂O was dissolved in 1 ml of water in a weighing dish, and then added to the perrhenate solution. 0.345 g of LiNO₃ was dissolved in 2 ml of water and added to the perrhenate solution. The perrhenate/lithium sulfate/lithium nitrate solution was allowed to stir, ensuring complete dissolution. This dopant solution was then added to 189.7 g of the above-prepared silver solution (specific gravity = 1.55 g/ml), and the resulting solution diluted with water to a total weight of 204 g. One-fourth of this solution was used to prepare a catalyst. 0.0923 g of stock CsOH solution containing 46.5 percent weight cesium was added to a 51 g portion of the silver oxalate/dopant solution to prepare the final impregnation solution.

The final impregnation solution thus prepared was then used to impregnate a cesium pre-doped carrier in the manner described below.

Approximately 30 g of the cesium pre-doped carrier described above for Catalyst F were placed under 3.33 kPa vacuum for 3 minutes at room temperature. Approximately 50 g of doped impregnating solution was then introduced to submerge the carrier, and the vacuum maintained at 3.33 kPa for an additional 3 minutes. At the end of this time, the vacuum was released, and excess impregnating solution removed from the carrier by centrifugation for 2 minutes at 500 rpm. Then the impregnated carrier was cured by being continuously shaken in an 8500 litre/hr air stream flowing across a cross-sectional area of approximately 19-32 cm² at 250-270 °C for 5-6 minutes. The cured cesium pre-doped catalyst (Catalyst F) was then ready for testing.

### Catalyst G (Rhenium-containing, no pre-doped Cs)

Catalyst G was prepared in the same manner as Catalyst F, except that the catalyst carrier was not pre-doped with cesium, and 0.128 g of the stock CsOH solution was added to a 51 g portion of silver stock solution.

### Catalyst H (Non-rhenium, pre-doped Cs)

For preparing impregnated Catalyst H, a solution was prepared by adding cesium hydroxide (0.3129 grams, 43.6% Cesium) to 350 g of the above-prepared silver oxalate/ethylenediamine solution (specific gravity = 1.553 g/ml).

The impregnation solution thus prepared was then used to impregnate a cesium pre-doped carrier in the manner described below.

Approximately 30 g of the cesium pre-doped carrier described in Part B above for Catalyst H were placed under 3.33 kPa vacuum for 3 minutes at room temperature. Approximately 50 g of the cesium-doped impregnating solution was then introduced to submerge the carrier, and the vacuum maintained at 3.33 kPa for an additional 3 minutes. At the end of this time, the vacuum was released, and excess impregnating solution removed from the carrier by centrifugation for 2 minutes at 500 rpm. The impregnated carrier was then cured by being continuously shaken in an 8500 litre/hr air stream at 250 °C for 5 minutes (over a cross-section area of approximately 19-32 cm²). The cured cesium pre-doped catalyst (Catalyst H) was then ready for testing.

### Catalyst I (Non-rhenium, no pre-doped Cs)

Catalyst I was prepared in the same manner as Catalyst H, except that the catalyst carrier was not pre-doped with cesium, and the amount of cesium hydroxide was adjusted to deposit 270 ppm of cesium on the finished catalyst.

The procedures set forth above for Catalysts F, G, H and I yielded catalysts on this carrier which contain approximately 13.5%w-14.5%w Ag with the following approximate dopant levels (expressed in parts per million by weight basis the weight of the total catalyst, i.e., ppmw) and which are approximately optimum in cesium for the given silver and rhenium, if present, and sulphur levels and support with regard to initial selectivity under the test conditions described below.

| | Cesium Pre-Doped, ppmw | Cs, ppmw | Re, ppmw | S, ppmw |
|---|---|---|---|---|
| Catalyst F | 345 | 420 | 280 | 48 |
| Catalyst G | 0 | 580 | 280 | 48 |
| Catalyst H | 510 | 200 | 0 | 0 |
| Catalyst I | 0 | 270 | 0 | 0 |

The actual silver content of the catalyst can be determined by any of a number of standard, published procedures. The actual level of rhenium on the catalysts prepared by the above process can be determined by extraction with water, followed by spectrophotometric determination of the rhenium in the extract. The actual level of cesium on the catalyst can be determined by employing a stock cesium hydroxide solution, which has been labelled with a radioactive isotope of cesium, in catalyst preparation. The cesium content of the catalyst can then be determined by measuring the radioactivity of the catalyst. Alternatively, the cesium content of the catalyst can be determined by leaching the catalyst with boiling deionized water. In this extraction process cesium, as well as other alkali metals, is measured by extraction from the catalyst by boiling 10 grams of whole catalyst in 20 millilitres of water for 5 minutes, repeating the above two more times, combining the above extractions and determining the amount of alkali metal present by comparison to standard solutions of reference alkali metals using atomic absorption spectroscopy (using Perkin Elmer Model 1100 B or equivalent).

### Part D: Catalyst Tests

### Part D.1: With catalysts A-C

3 to 5 grams of crushed catalyst (1.41-0.84 mm, i.e. 14-20 mesh) were loaded into a ¼ inch diameter stainless steel U-shaped tube. The U tube was immersed in a molten metal bath (heat medium) and the ends connected to a gas flow system. The weight of the catalyst used and the inlet gas flow rate were adjusted to achieve a gas hourly space velocity of 3300 ml of gas per ml of catalyst per hour. The inlet gas pressure is 1550 kPa.

The gas mixture passed through the catalyst bed (in once-through operation) during the entire test run (including start-up) consisted of 30% ethylene, 8.5% oxygen, 5% carbon dioxide, 54.5% nitrogen, and 2.0 to 6.0 ppmv ethyl chloride.

The initial reactor (heat medium) temperature was 225 °C. After 1 hour at this initial temperature, the temperature was increased to 235 °C for 1 hour, and then adjusted to 245 °C for 1 hour. The temperature was then adjusted so as to achieve a constant oxygen conversion level of 40%. Performance data at this conversion level were usually obtained when the catalyst has been on stream for a total of at least 1-2 days. Due to slight differences in feed gas composition, gas flow rates, and the calibration of analytical instruments used to determine the feed and product gas compositions, the measured selectivity and activity of a given catalyst may vary slightly from one test run to the next. To allow meaningful comparison of the performance of catalysts tested at different times, all catalysts described in this illustrative embodiment were tested simultaneously with a reference catalyst. All performance data reported in this illustrative embodiment are corrected relative to the average initial performance of the reference catalyst (S₄₀ = 81.0%; T₄₀ = 230 °C).

The results are presented below in Table II. All selectivity values are expressed as %.

**TABLE II**

| Initial Performance of Catalyst (Data Obtained at 40% Conversion) | |
|---|---|
| Catalyst | Initial Selectivity |
| A(Li) | 82.0% |
| B(K) | 81.8% |
| C | 81.0% |

As can be seen from Table II, the catalyst which was prepared using a pre-doped lithium carrier (Catalyst A), had a higher initial selectivity than a catalyst prepared without using a pre-doped potassium carrier (Catalyst C), and was thus advantaged. It can also be seen than the catalyst which was prepared using a pre-doped potassium carrier (Catalyst B), had a higher initial selectivity than a catalyst prepared without using a pre-doped lithium carrier (Catalyst C), and was thus advantaged.

### Part D.2: With catalysts D and E

The initial conditions were as described above for catalyst A-C.

After obtaining initial performance values for selectivity at 40% conversion the catalysts were subjected to high severity aging conditions. Under these conditions, the catalyst was heated to either 85% conversion or a maximum of 285 °C for a 10-day period to accelerate the aging of the catalyst. After this 10-day aging period, the catalysts were again brought to 40% conversion and reoptimized under standard conditions. The selectivity was again measured, and compared to the original value of the fresh catalyst. After the new selectivity value was determined, this cycle was repeated, and the selectivity decline of the catalyst continuously measured under standard 40% conversion conditions in 10-day cycles relative to its fresh initial performance. The results are presented below in Table III. All selectivity values are expressed as %. The initial performances of Catalysts D and E were determined to be the same, within experimental error. Initial S₄₀ values of 86.4% ± 0.4% and T₄₀ values of 264 °C ± 3 °C were obtained.

**TABLE III**

| Loss in Selectivity* from Fresh Catalyst | | | | | | | |
|---|---|---|---|---|---|---|---|
| Total Days at Either 85% Conversion or 285 °C (Data Obtained at 40% Conversion Conditions) | | | | | | | |
| Catalyst | 10 Days | 20 Days | 30 Days | 40 Days | 50 Days | 60 Days | 70 Days |
| D(Li) | -0.0% | -0.2% | -1.4% | -2.7% | -3.7% | -4.3% | -5.1% |
| E | -1.3% | -2.5% | -3.4% | -4.1% | -5.1% | -6.3% | -7.4% |

| | | | | | | | |
|---|---|---|---|---|---|---|---|
| * Loss in Selectivity (%) = { S_{4O}, % (Aged) } - {S_{4O}, % (Fresh) } | | | | | | | |

As can be seen from Table III, Catalyst E, which was prepared without using a pre-doped lithium carrier, decreased in selectivity much more rapidly than did Catalyst D which was prepared using a pre-doped lithium carrier.

### Part D.3: With catalysts F and G

The initial conditions were as described above for catalyst A-C, except that the amount of crushed catalyst used was 1.5-2 grams, the gas hourly space velocity 6800, and the constant ethylene oxide production level 1.5%.

1.5 to 2 grams of crushed catalyst (0.841-0.595 mm, i.e. 20-30 mesh) were loaded into a ¼ inch diameter stainless steel U-shaped tube.

The gas mixture passed thorough the catalyst bed (in once-through operation) during the entire test run (including start-up) consisted of 25% ethylene, 7% oxygen, 5% carbon dioxide, 63% nitrogen, and 1.0 to 6.0 ppmv ethyl chloride.

All selectivity data reported in this illustrative embodiment are corrected relative to the average initial performance of the reference catalyst (S_{1.5} = 81.7%).

After obtaining initial performance values for selectivity at 1.5% ethylene oxide production the catalysts were subjected to high severity aging conditions. After initial performance values were determined, Catalysts F and G were tested for an extended period of time to determine the stability of the catalysts.

The results are presented in the following Table IV.

**TABLE IV**

| Loss of Selectivity* from Fresh Catalyst Cumulative Ethylene Oxide Production (kg Ethylene Oxide/litre Catalyst) | | | | | | | |
|---|---|---|---|---|---|---|---|
| Catalyst | 80 | 160 | 240 | 320 | 400 | 480 | 560 |
| F (Re/Cs) | -0.6 | -1.9 | -2.3 | -2.6 | -3.8 | -3.8 | -4.3 |
| G (Re) | -0.9 | -1.6 | -2.2 | -5.0 | -5.7 | -6.3 | -6.6 |

| | | | | | | | |
|---|---|---|---|---|---|---|---|
| * Loss of Selectivity (%) = { S_{1.5}, % (Aged) } - {S_{1.5}, % (Fresh) } | | | | | | | |

### Part D.4: With catalysts H and I

The initial conditions were as described above for catalysts A-C. All performance data reported in this illustrative embodiment are corrected relative to the average initial performance of the reference catalyst (S₄₀ = 81.0%; T₄₀ = 230 °C). The initial performances of Catalysts F and G were determined to be the same, within experimental error.

After obtaining initial performance values for selectivity at 40% conversion the catalysts were subjected to high severity aging conditions. The high severity testing was done under the following conditions: 30,000 GHSV, 7% CO₂, 8.5% O₂, 30% ethylene, 5.6 ppm EC, balance N₂. Reactor pressure was 1550 kPa and the oxygen conversion was kept constant at 25%. The results are presented below in Table V. All selectivity values are expressed as %.

**TABLE V**

| Loss of Selectivity* from Fresh Catalyst Total Days Under High Severity Testing Conditions (Data Obtained at 25% Conversion Conditions) | | | | |
|---|---|---|---|---|
| Catalyst | 1 Day | 10 Days | 20 Days | 30 Days |
| H (Cs) | 0% | 0% | -1% | -1.5% |
| I | 0% | 0% | -1.8% | -2.3% |

| | | | | |
|---|---|---|---|---|
| * Loss in Selectivity (%) = { S₂₅, % (Aged) } - {S₂₅, % (Fresh) } | | | | |

As can be seen from Tables IV and V, catalysts which were prepared using a pre-doped cesium carrier, both the rhenium-containing catalyst (Catalyst F) and the non-rhenium containing catalyst (Catalyst H), declined less rapidly than rhenium-containing and non-rhenium containing catalysts prepared without using a pre-doped cesium carrier, Catalysts G and I respectively, and were thus significantly advantaged.

## Claims

1. A process for preparing a catalyst for the vapour phase production of ethylene oxide from ethylene and oxygen which process comprises depositing a predopant amount of potassium, on a shaped porous refractory support, in salt, compound or potassium complex form which has been dissolved in an aqueous or essentially aqueous solution, drying the support to a degree sufficient to fix the potassium on the support before deposition of the other catalyst components at a temperature in the range of from 200°C to 1000°C and thereafter depositing a catalytically effective amount of silver, a promoting amount of alkali metal and a promoting amount of rhenium on said support, and optionally a promoting amount of a rhenium co-promoter selected from sulphur, molybdenum, tungsten, chromium, phosphorus, boron and mixtures thereof, and thereafter drying the support.

2. The process of claim 1 wherein the alkali metal predopant is a potassium compound, which is selected from potassium acetate, potassium nitrate, potassium chloride, potassium oxide, potassium hydroxide, potassium oxalate and mixtures thereof.

3. The process of claim 1, wherein the support comprises alpha alumina, having a surface area in the range of from 0.05 m²/g to about 10 m²/g.

4. The process of claim 1 wherein the alkali metal promoter is selected from potassium, rubidium, cesium and mixtures thereof.

5. The process of claim 4 wherein the alkali metal promoter comprises cesium plus at least one additional alkali metal.

6. The process of claim 5 wherein the alkali metal promoter is cesium plus lithium.

## Patentansprüche

1. Verfahren zur Herstellung eines Katalysators zur Herstellung von Ethylenoxid aus Ethylen und Sauerstoff in der Dampfphase, welches Verfahren ein Ablagern einer vordotierenden Menge von Kalium auf einem geformten, porösen, feuerfesten Träger, in Form eines Salzes, einer Verbindung oder eines Kaliumkomplexes, welche in einer wässrigen oder im wesentlichen wässrigen Lösung gelöst ist, ein Trocknen des Trägers in einem ausreichenden Ausmaß, um das Kalium auf dem Träger vor der Ablagerung der anderen Katalysatorkomponenten bei einer Temperatur im Bereich von 200°C bis 1000°C zu fixieren, und anschließend ein Ablagern einer katalytisch wirksamen Menge von Silber, einer Promotormenge von Alkalimetall und einer Promotormenge von Rhenium und gegebenenfalls einer Promotormenge eines Rhenium-Copromotors, ausgewählt unter Schwefel, Molybdän, Wolfram, Chrom, Phosphor, Bor und Gemischen hievon, auf dem Träger und ein anschließendes Trocknen des Trägers umfaßt.

2. Verfahren nach Anspruch 1, worin das Alkalimetall-Vordotierungsmittel eine Kaliumverbindung ist, die unter Kaliumacetat, Kaliumnitrat, Kaliumchlorid, Kaliumoxid, Kaliumhydroxid, Kaliumoxalat und deren Gemischen ausgewählt ist.

3. Verfahren nach Anspruch 1, worin der Träger alpha-Aluminiumoxid mit einer Oberfläche im Bereich von 0,05 m²/g bis etwa 10 m²/g umfaßt.

4. Verfahren nach Anspruch 1, worin der Alkalimetallpromotor unter Kalium, Rubidium, Cäsium und deren Gemischen ausgewählt ist.

5. Verfahren nach Anspruch 4, worin der Alkalimetallpromotor Cäsium und wenigstens ein weiteres Alkalimetall umfaßt.

6. Verfahren nach Anspruch 5, worin der Alkalimetallpromotor Cäsium plus Lithium ist.

## Revendications

1. Procédé pour préparer un catalyseur pour la production en phase vapeur d'oxyde d'éthylène à partir d'éthylène et d'oxygène, lequel procédé comprend les étapes consistant à déposer une quantité pré-dopante de potassium sur un support réfractaire poreux façonné, sous la forme d'un sel, d'un composé ou d'un complexe de potassium qui a été dissous dans une solution aqueuse ou essentiellement aqueuse, sécher le support à un degré suffisant pour fixer le potassium sur le support avant le dépôt des autres composants du catalyseur à une température dans la plage de 200 °C à 1000 °C, déposer ensuite une quantité d'argent efficace au plan catalytique, une quantité promotrice de métal alcalin et une quantité promotrice de rhénium sur ledit support, et éventuellement une quantité promotrice d'un co-promoteur de rhénium choisi parmi le soufre, le molybdène, le tungstène, le chrome, le phosphore, le bore et leurs mélanges et ensuite sécher le support.

2. Procédé suivant la revendication 1, dans lequel le prédopant de métal alcalin est un composé de potassium, qui est choisi parmi l'acétate de potassium, le nitrate de potassium, le chlorure de potassium, l'oxyde de potassium, l'hydroxyde de potassium, l'oxalate de potassium et leurs mélanges.

3. Procédé suivant la revendication 1, dans lequel le support comprend de l'alpha-alumine, ayant une aire superficielle allant de 0,05 m²/g à environ 10 m²/g.

4. Procédé suivant la revendication 1, dans lequel le promoteur de métal alcalin est choisi parmi le potassium, le rubidium, le césium et leurs mélanges.

5. Procédé suivant la revendication 4, dans lequel le promoteur de métal alcalin comprend du césium plus au moins un métal alcalin additionnel.

6. Procédé suivant la revendication 5, dans lequel le promoteur de métal alcalin est du césium plus du lithium.
